# EUROPEAN PATENT APPLICATION

(11) **EP 2 929 873 A1**
(43) Date of publication of application: **14.10.2015**
(21) Application number: 14001271.7
(22) Date of filing: 07.04.2014
(51) Int. Cl.: A61K 8/49, A61K 8/73, A61Q 17/00, A61Q 19/00, A61Q 19/10, A61K 8/97, A61K 36/87, A61K 36/889, A61K 36/15, A61K 36/45, A61K 36/54, A61K 36/73

(54) **Skin cleansing compositions**

(71) Applicant: Intermed S.A., 145 64 Kifissia (GR)
(72) Inventor: Tseti, Ioulia, 145 61 Kifissia (GR)
(74) Representative: Wibbelmann, Jobst

(57) **Abstract**

The present invention relates to an aqueous skin cleansing composition comprising at least one proanthocyanidine, an α-Glucan oligosaccharide, and a surfactant, and a tissue or cloth impregnated with this composition.

## Description

The present invention concerns a composition for skin cleansing. The composition is useful for cleansing the peri-genital skin area and the external genitalia of women and men under conditions that may support microbial growth in the vagina or urinary tract like, for example, sexual intercourse, swimming and bathing, intense physical action or menopause. The composition is particularly useful against Escherichia coli infections. It is a combination of a proanthocyanidine containing material of botanical origin (like apple, maritime pine, cinnamon, aronia, cocoa, red grapes, bilberry, cranberry, black currant, green tea, black tea and acai) and a carbohydrate derivative and it may be presented as a solution, soap, liquid soap, emulsion or impregnated wipes.

### Prior art

Preparations for treatment and/or prevention of urogenital infections containing proanthocyanidines of botanical origin (alone or in combination with carbohydrates) have been described in the art as early as 1986. Patent application DE3427014 describes a synergistic blend of cranberry juice, fructose, vitamin C and magnesium that reduces the adhesiveness of typical pathogens of urinary tract infections to the transitional epithelium of the urinary tract. WO2004056380 is a composition containing a plant extract from the Ericaceae, Rosaceae, Pinaceae or Vitaceae family and a sugar that is not metabolized or it is partially metabolized by the human body. According to claim 32 of WO2004056380 the composition is used for treating or preventing bacterial infection. WO2009089442 is about "a method of preventing, controlling and ameliorating urinary tract infections using a synergistic cranberry derivative and d-mannose composition". WO03086274 describes a compound for cleansing the skin of men and women before, during and after sexual intercourse. Cranberry extract is contained in the product that is presented as a tissue, soap and cream.

### The problem

Escherichia coli is a Gram-negative, facultative anaerobic, rod-shaped bacterium that is commonly found in the lower intestine of warm-blooded organisms. Most E. coli strains are harmless including those normally forming part of the normal human gut flora. Virulent strains, though, are responsible for a number of human diseases including urinary tract infections and bacterial vaginitis. E. coli has been proven to be responsible for the vast majority of recurrent (antibiotic treatment resistant) episodes of urogenital infections due to its ability to adhere to epithelium surfaces and resist antibiotic therapies.

Urogenital tract infections (UTIs) are very common. 50 to 60% of women experience a UTI during their lifetime. Recurrent UTI is defined as 2 uncomplicated UTIs in 6 months or, more traditionally, as 3 positive cultures within the preceding 12 months. This is estimated to affect 25% of women with a history of UTI. When there is recurrent infection with the same organism despite adequate therapy, it is considered a relapse. Reinfection is defined as recurrent UTI caused by a different bacterial isolate, or by the previously isolated bacteria after a negative intervening culture or an adequate time period (2 weeks) between infections.

Even though reinfections are more common than relapses, when the initial infection is caused by E. coli, there is a higher risk of reinfection within the first 6 months.

Ampicillin and sulfonamides cannot be used for empiric therapy of E. coli infections of the urogenital epithelium since more than one third of isolates demonstrate in vitro resistance. More than 15% to 20% of E. coli strains causing uncomplicated cystitis are now resistant to these agents. Other agents like nitrofurantoin and fluoroquinolones are still effective on E. coli uropathogens but cannot be used on multi-agent infections since many non-E. coli pathogens that usually are present in UTI infections are resistant to these agents.

3-day regimens of trimethoprim, trimethoprimsulfamethoxazole, or a fluoroquinolone for treatment of acute uncomplicated cystitis, are currently recommended for empiric therapy. Nitrofurantoin is a safe and generally effective agent, but it should be administered for a minimum of 7 days. Single-dose regimens are somewhat less effective than 3- to 7-day regimens, even with fluoroquinolones. First-line treatment suggested by the Infectious Disease Society of America in 1999 was TMP-SMX in a 3-day regimen. Current data suggest high TMP-SMX resistance among uropathogens.

Given the high prevalence of recurrent E. coli UTIs and the high health and economical risks related to multi-agent and/or multi-dose regimens associated with treatment there has been an increasing need for alternative, complementary or preventive measures.

The skin cleansing compositions described herein provide such solutions.

### The invention

With the above aims and problems in mind, the compositions as described herein have been developed. The compositions comprise:
- A proanthocyanidine containing material of botanical origin. Useful plant parts that can be used as a source for such a material include: apple fruits, maritime pine bark, cinnamon bark, aronia fruit, cocoa beans, grape seed, red grape skin, red wine, bilberry fruit, cranberry fruit, black currant fruit, green tea leaves, black tea leaves and acai oil. Such a materials may be used as they are and, also, as dry and liquid extracts and distillates.
- One or more oligosaccharides.
- A vehicle suitable for the combined skin application of the proanthocyanidine and oligosaccharides materials in liquid, semi-liquid or solid form which contain: water, one or more surfactants (non-ionic, anionic, cationic or amphoteric), a suitable preservative system, an acidifier in order to provide a pH suitable close to the one of the vaginal mucosa (preferably, but not limited to, lactic acid).
- The above vehicle may also contain: a suitable thickener, one or more vitamins (pro-vitamin forms may also be used), vegetable derived oil materials.
- A non-woven impregnation medium can be for application of the composition.

A suitable source for proanthocyanidines include cranberries. Cranberries have a complex composition and particularly comprise flavan-3-ols, A-type procyanidines (PACs), anthocyanines, benzoic acid, and ursolic acid. The flavan-3-ols that are found in cranberries can be present as monomers, oligomers, and polymers. The oligomers and polymers are also referred to as PACs or condensed tannins. They represent about 85% of the total flavan-3-ols on a weight basis.

Generally, PACs can be included in compositions that comprise an extract from a plant that is a member of the ericaceae, rosaceae, pinaceae or vitaceae family. Thus, extracts from plants bearing berries are preferred and particularly include those that are red, blue or purple in colour, such as, for example, blueberries, bilberries, cowberries, cranberries, crowberries, farkleberries, lingonberries, partridgeberries, huckleberries, whortleberries or sparkleberries. In a particularly preferred embodiment, the plant is cranberry or bilberry. In the context of the present invention, the proanthocyanidine can be typically provided in the form of an extract from the plant.

The plant extract may be prepared by conventional extraction techniques, such as drying, grinding, pressing, pulping, pulverisation, lyophilisation, solvent and extraction, steam distillation, and the like. Preferably, the extract is in the form of a juice or concentrate. In a particularly preferred embodiment, the plant extract is an extract of cranberries. Suitable cranberry extracts may be prepared, for example, by methods such as disclosed in WO 99/12541 and WO 03/084559.

The extract may further comprise at least one polyphenolic compound, preferably a flavonoid compound selected from the group consisting of anthocyanidines, proanthocyanidines, flavanols, flavones, flavanones, and isoflavanones. In particular, the flavonoid compound is, preferably, an anthocyanidin or a proanthocyanidin. The second ingredient of particular importance in the context of the present invention, in particular when it is important to treat or even prevent the occurrence of infections due to the presence of E.coli, is the oligosaccharide.

It has been found by the inventors that in combination the oligosaccharides with some microbial efficacy can be advantageously employed. One particular example include oligosaccharides obtainable from natural sugars such as sucrose and maltose which are defined as alpha-glucan oligosaccharides. A particular preferred example includes oligo(1,4-alpha-glucan). These oligosaccharides may comprise from 2 to 9 monomeric glucose and/or fructose units as well as other "sugar" units well-known to the skilled person.

The composition of the present invention is particularly suitable to impregnate a tissue or cloth which can then be used for the purposes of the present invention. Such use include *inter alia* methods for preventing urogenital tract infection by applying the tissue or cloth on perigenital skin area. This application may include the cleansing of the perigenital skin as well as a simple prevention activity by wiping the tissue and/or cloth across the perigenital skin.

The use of cloth and/or tissue helps to improve the feeling of freshness and personal hygiene, in particular after sexual intercourse. After a sexual intercourse, the tissue may be provided to each participant. This tissue may be impregnated with the composition according to the present invention, such that the tissue is considered a wet tissue.

The tissue or cloth may be a fabric that is cut into square or rectangular sheets (wipes) of approximately 20 cm long by 25 cm wide, just to give an example. Each sheet may be folded and packed in a hermetic sachet, so as to prevent evaporation and drying of the liquid or gel-like composition.

The composition certainly includes various other ingredients, such as surfactants. These surfactants include various singular compositions as well as mixtures. The surfactant may be *inter alia* an anionic surfactant, such as disodium laureth sulfosuccinate and/or an non-ionic surfactant, such as decyl glucoside.

### Detailed Description of Preferred Embodiments

Several examples of skin cleansing compositions in accordance with the above general description of the invention are presented in the following embodiments:

### First embodiment

Example 1 (Table 1) describes a liquid soap composition intended to cleanse the perigenital skin area and the external genitalia of women and men under conditions that may support microbial growth in the vagina or urinary tract like, for example, sexual intercourse, swimming and bathing, intense physical action or menopause. This embodiment is an aqueous solution of:
- Cranberry fruit extract as the proanthocyanidine source.
- Alpha glucan oligosaccharide as the carbohydrate derivative of choice.
- Lactic acid as a pH adjuster. The final pH of the composition should be adjusted at < 5.5 in order to provide optimum microbial stability and compatibility.
- A proprietary blend of levulinic and anisic acid, marketed under the trade name Dermosoft 1388 by Dr. Straetmans GmbH, as a preservative system.
- A surfactant system combining an anionic (disodium laureth sulfosuccinate) and a non-ionic (decyl glucoside) surfactant.
- PEG-150 distearate and laureth-2 as the thickener system.
- Several skin conditioning agents combining water (panthenol and PEG-7 glyceryl cocoate) and lipid (alpha bisabolol, rosa moschata oil and borago officinalis seed oil) soluble materials.
- A proprietary blend of tocopherol and ascorbyl palmitate, marketed under the trade name Controx VP by Cognis Care Chemicals, as an antioxidant system.

For the preparation of 100 L of the above described liquid soap composition the purified water needed for the batch is transferred into a suitable processing unit having planetary mixing and steam heating capabilities. After dissolution of the surfactants, the solution is heated up to 75°C and PEG-150 distearate is melted into the bulk. The remaining materials are added upon cooling under 45°C and under continuous stirring to the mix. After pH adjustment with lactic acid at 4.5 the batch is cooled down to 25°C.

### Second embodiment

Example 2 (Table 1) describes a low viscosity foaming solution composition intended to cleanse the perigenital skin area and the external genitalia of women and men under conditions that may support microbial growth in the vagina or urinary tract like, for example, sexual intercourse, swimming and bathing, intense physical action or menopause. The solution is designed for used with a foaming pump apparatus.

Its design characteristics and manufacturing method are similar to the ones described for the first embodiment. Since no thickener is utilized in this second embodiment the high heating step to 75°C should be omitted.

### Third embodiment

Example 3 (Table 1) describes a low viscosity & low foaming solution composition intended to cleanse the perigenital skin area and the external genitalia of women and men under conditions that may support microbial growth in the vagina or urinary tract like, for example, sexual intercourse, swimming and bathing, intense physical action or menopause. The solution is designed for wet wipes impregnation.

Its design characteristics and manufacturing method are similar to the ones described for the first embodiment. Since no thickener is utilized in this second embodiment the high heating step to 75°C should be omitted.

**Table 1: Skin cleansing compositions**

| **Ingredient** | **Content (kg/100 L)** | | |
|---|---|---|---|
| | **Example 1** | **Example** 2 | **Example** 3 |
| Purified Water | Q.S. to 100 L | Q.S. to 100 L | Q.S. to 100 L |
| Cranberry Fruit Extract | 2.00 | 2.00 | 2.00 |
| Alpha Glucan Oligosaccharide | 2.00 | 2.00 | 2.00 |
| Disodium Laureth Sulfosuccinate | 20.00 | 20.00 | - - - |
| Decyl Glucoside | 8.00 | 8.00 | 2.00 |
| PEG-150 Distearate | 2.50 | - - - | - - - |
| PEG-7 Glyceryl Cocoate | 6.00 | 6.00 | 6.00 |
| Alpha Bisabolol | 0.10 | 0.10 | 0.10 |
| Rosa Moschata Oil | 0.25 | 0.25 | 0.25 |
| Borago Officinalis Seed Oil | 0.25 | 0.25 | 0.25 |
| Controx VP (Cognis Care Chemicals) | 0.05 | 0.05 | 0.05 |
| Laureth-2 | 1.00 | 1.00 | 1.00 |
| Dermosoft 1388 (Dr. Straetmans GmbH) | 3.00 | 3.00 | 2.00 |
| Methylisothiazolinone 10% | - - - | - - - | 0.10 |
| Lactic Acid 90% | Q.S. to pH = 4.50 | Q.S. to pH = 4.50 | Q.S. to pH = 4.50 |

## Claims

1. An aqueous skin cleansing composition comprising
(a) at least one proanthocyanidine,
(b) an α-glucan oligosaccharide, and
(c) a surfactant.

2. The composition according to claim 1, wherein the at least one proanthocyanidine is provided by a botanical material.

3. The composition according to claim 2, wherein the botanical material is selected from the group consisting of apple fruit extract, maritime pine bark extract, cinnamon bark extract, aronia fruit extract, cocoa beans extract, grape seed extract, red grape skin extract, red wine extract, bilberry fruit extract, cranberry fruit extract, black currant fruit extract, green tea leaves extract, black tea leaves extract and acai oil.

4. The composition to any one of claims 1 to 3, wherein the surfactant is selected from the group consisting of non-ionic, anionic, cationic and amphoteric surfactants.

5. The composition to any one of the preceding claims, wherein the composition comprises at least one preservative.

6. The composition according to claim 5, wherein the preservative is selected from the group consisting of benzoic acid, sorbic acid, anisic acid and levulinic acid or any sodium or potassium salt of the above acids.

7. The composition according to any one of the preceding claims having a pH between 3.5 and 5.5.

8. The composition according to any one of the preceding claims comprising lactic acid.

9. A tissue or cloth impregnated with a composition according to any one of the preceding claims.
